# FASCICULE DE BREVET EUROPEEN

(11) **EP 0 841 876 B1**
(45) Date de publication et mention de la délivrance du brevet: **31.07.2002**
(21) Numéro de dépôt: 95911352.3
(22) Date de dépôt: 27.02.1995
(51) Int. Cl.: A61B 17/58

(54) **CONNECTEUR POUR INSTRUMENTATION D'OSTEOSYNTHESE RACHIDIENNE, DESTINE A UNE FIXATION LOMBAIRE OU SACREE OU ILIO-SACREE**
VERBINDER ZUM GEBRAUCH FÜR WIRBELSÄULENOSTEOSYNTHESE-INSTRUMENTE ZUR LENDEN-, SAKRAL ODER ILIOSAKRAL- BEFESTIGUNG
CONNECTOR USED IN SPINAL OSTEOSYNTHESIS EQUIPMENT FOR LUMBAR, SACRAL OR ILIOSACRAL FIXATION

(30) Priorité: 03.03.1994 FR 9402473
(43) Date de publication de la demande: 20.05.1998
(73) Titulaire: SOCIETE DE FABRICATION DE MATERIEL ORTHOPEDIQUE SOFAMOR, 93290 Tremblay-en-France (FR)
(72) Inventeur: STEIB, Jean-Paul, F-67100 Strasbourg (FR)
(74) Mandataire: Moncheny, Michel
(86) Numéro de dépôt international: FR9500230
(87) Numéro de publication internationale: WO9523559

(56) Documents cités:
- EP-A- 0 384 001
- EP-A- 0 443 894
- EP-A- 0 468 264
- WO-A-94/00062
- WO-A-94/10928

## Description

La présente invention a pour objet un connecteur pour instrumentation d'ostéosynthèse rachidienne, destiné à une fixation lombaire, ou sacrée ou ilio-sacrée, adapté pour solidariser une tige d'ostéosynthèse avec une vis d'ancrage osseux.

Ce connecteur comporte un corps percé d'un alésage cylindrique de passage de ladite tige, et d'un trou taraudé débouchant perpendiculairement dans cet alésage, ce trou étant adapté pour recevoir un élément de fixation (vis) de la tige au connecteur. Celui-ci comprend en outre au moins une lame monopièce avec le corps et le prolongeant latéralement, au moins un trou de passage d'une vis d'ancrage osseux étant réalisé dans la lame.

On connaît par le brevet français 2 657 774 (9001474) un connecteur constitué par un sabot de prise sacrée de ce type, la ou les lame(s) s'étendant sensiblement dans le prolongement de l'une des surfaces du corps. On connaît également une instrumentation d'ostéosynthèse pour la correction du spondylolisthésis par voie postérieure, qui comprend des connecteurs répondant à la définition ci-dessus, et dont par conséquent l'une des surfaces qui délimite la lame est contenue dans le même plan que celui de la surface plane contigüe du corps du connecteur.

On utilise enfin pour des fixations sacro-iliaques des connecteurs d'extension dits "de Chopin" ayant une configuration particulière. Chacun de ces trois types de connecteurs possède une conformation propre, adaptée à son application.

Cependant, ces trois exemples de connecteurs connus ne sont pas limitatifs, la plupart des implants de ce type ayant une forme spécifiquement destinée à une pathologie déterminée. Il en résulte qu'une instrumentation comme celle connue sous la marque commerciale "CCD" (Compact Cotrel Dubousset) comprend un nombre élevé d'implants différents.

De plus, chacun de ces connecteurs spécifiques ne peut être placé indifféremment à gauche ou à droite du montage vertébral, de sorte que pour chaque connecteur il faut en outre prévoir un connecteur gauche et un connecteur droite.

Certains des connecteurs connus, par exemple le sabot de Chopin, présentent un encombrement parfois gênant. De même le document EP-A-0 468 264 décrit un connecteur dont la lame est agencée de telle sorte que, en position d'utilisation, le connecteur présente un encombrement important au-dessus de la surface osseuse, susceptible de constituer une gêne appréciable pour le patient. Enfin, il subsiste souvent, comme dans le connecteur du document précité, un tronçon plus ou moins long de la vis d'ancrage osseux qui n'est pas enfoncé dans le corps vertébral, et qui ne participe donc pas effectivement à la solidité de l'ancrage.

L'invention a pour but de réaliser un connecteur agencé de façon à éliminer ces divers inconvénients, soit complètement, soit dans une très large mesure.

Conformément à l'invention, le connecteur est caractérisé en ce que la lame présente deux surfaces latérales planes sensiblement parallèles qui s'étendent de part et d'autre d'un plan contenant l'axe de l'alésage et parallèle à ces deux surfaces latérales, lesquelles sont raccordées par des décrochements aux surfaces contigües du corps.

Ainsi, la lame ou du moins l'une de ses surfaces latérales, ne s'étend plus dans le même plan que le plan contenant la surface plane contigüe du corps, comme dans certains connecteurs antérieurs, mais est décalée par rapport à cette surface plane latérale du corps, vers l'axe de l'alésage.

On constate qu'avec une telle configuration, le même connecteur peut être utilisé de manière pratiquement universelle pour les différentes applications mentionnées ci-dessus, ce qui présente un avantage considérable. En effet, une seule pièce, qui de plus peut être utilisée indifféremment à droite ou à gauche, peut remplacer les connecteurs connus jusqu'à présent pour la fixation sacrée, la fixation ilio-sacrée, et la réduction-fixation du spondylolisthèsis.

Suivant une caractéristique de l'invention, la lame s'étend de manière asymétrique par rapport au plan contenant l'axe de l'alésage, la surface latérale opposée au bord d'entrée du trou d'introduction de la vis étant plus proche dudit plan que l'autre surface latérale.

Etant donné que l'alésage de passage de la tige d'ostéosynthèse n'est pas percé dans la zone médiane du corps, mais décalé latéralement pour réserver à la vis de fixation un trou taraudé d'une profondeur suffisante, l'ensemble du connecteur présente une configuration asymétrique nettement moins encombrante que les connecteurs antérieurs, en particulier les sabots de fixation sacrée.

D'autres particularités et avantages de l'invention apparaîtront au cours de la description qui va suivre, faite en référence aux dessins annexés qui en illustrent plusieurs formes de réalisation à titre d'exemples non limitatifs.

La figure 1 est une vue en élévation latérale, à échelle agrandie, d'un premier mode de réalisation du connecteur pour instrumentation d'ostéosynthèse rachidienne selon l'invention.

La figure 2 est une vue de dessus du connecteur de la figure 1.

La figure 3 est une vue en élévation latérale suivant la direction K de la figure 1.

La figure 4 est une vue en élévation et coupe partielle, à échelle agrandie, du connecteur des figures 1 à 3 ainsi que de la tige d'ostéosynthèse et d'une vis d'ancrage solidarisées par ce connecteur, la vis étant enfoncée dans un corps osseux.

Les figures 5 et 6 sont des vues en élévation partielle à échelle agrandie de l'extrémité d'une tige d'ostéosynthèse avant montage sur celle-ci d'un connecteur selon l'invention (figure 5), et au début de l'opération de montage de ce connecteur (figure 6) après soulèvement de l'extrémité de la tige.

La figure 7 est une vue en perspective éclatée d'un connecteur selon l'invention, de l'extrémité d'une tige d'instrumentation d'ostéosynthèse rachidienne et d'une vis d'ancrage en position d'introduction dans le connecteur.

La figure 8 est une vue en élévation en bout de l'instrumentation de la figure 7 fixée sur le sacrum.

La figure 9 est une vue en perspective partielle d'une fixation lombaire avec un connecteur selon les figures 1 à 6, l'instrumentation étant destinée à la réduction du spondylolisthèsis.

Les figures 10 et 11 sont des vues respectivement en élévation à échelle agrandie et de dessus d'un écrou conique pouvant être introduit dans le trou de la lame du connecteur de l'instrumentation de la figure 9 et coopérer avec une vis à double filetage d'ancrage osseux.

La figure 12 est une vue en perspective d'une fixation ilio-sacrée incorporant un connecteur selon les figures 1 à 6.

La figure 13 est une vue en perspective d'une seconde forme de réalisation du connecteur selon l'invention, ainsi que de la tige et de la vis d'ancrage osseux correspondant.

La figure 14 est une vue en élévation d'une troisième forme de réalisation du connecteur selon l'invention, fixé à une vertèbre lombaire, ainsi que de la tige d'ostéosynthèse correspondante.

- Les figures 15 à 18 illustrent la méthode chirurgicale de pose du connecteur selon l'invention en fixation sacrée.

- Les figures 19 et 20 sont des vues illustrant la méthode chirurgicale de pose du connecteur selon l'invention en fixation iliaque.

La figure 21 est une vue en perspective illustrant la méthode de pose d'un connecteur selon l'invention en fixation lombaire décalée lorsque la vis à double filetage n'est pas encore en place.

La figure 22 est une vue en perspective illustrant la méthode de pose d'un connecteur selon l'invention en fixation lombaire décalée lorsque la vis à double filetage est déjà en place.

La figure 23 est une vue en perspective illustrant le montage en fixation lombaire décalée d'un connecteur selon l'invention.

Les figures 24 et 25 sont des vues en élévation d'un segment vertébral illustrant deux méthodes possibles de pose du connecteur en fixation antérieure.

Le connecteur représenté aux dessins est destiné à faire partie d'une instrumentation d'ostéosynthèse rachidienne lombaire, notamment pour la réduction-fixation du spondylolisthèsis, ou sacrée, ou ilio-sacrée.

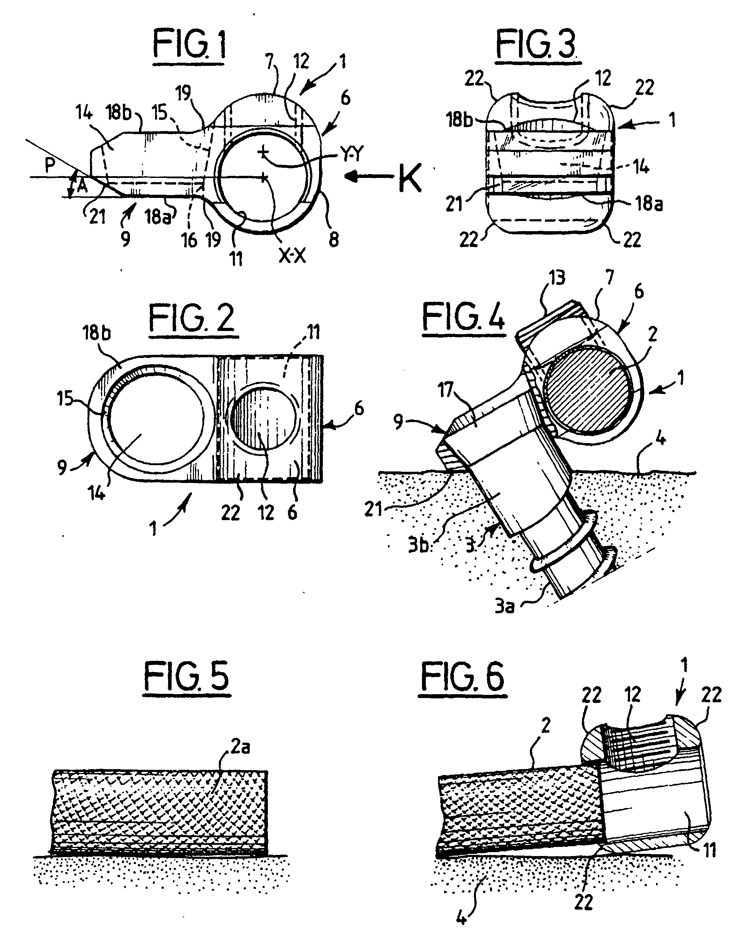

Le connecteur 1 représenté aux figures 1 à 6 est adapté pour pouvoir solidariser une tige d'ostéosynthèse 2 avec une vis 3 d'ancrage osseux dans un os 4 (vertèbre lombaire, sacrum ou os iliaque). La tige 2 est normalement cylindrique et de préférence du type à aspérités 2a constituées par exemple de pointes de diamant ou d'un moletage (tige Cotrel-Dubousset).

Le connecteur 1 est constitué d'un corps 6 de forme oblongue vu en section transversale, avec deux surfaces opposées 7, constituées de portions de cylindre, reliées d'un côté par une surface sensiblement plane 8, ainsi que par une lame 9 du côté opposé. Le corps 6 est percé d'un alésage cylindrique 11 de passage de la tige d'ostéosynthèse 2 et dont l'axe XX est décalé vers l'une des surfaces latérales 7, par rapport à l'axe YY du corps 6, et parallèlement à cet axe. Le corps 6 est également percé d'un trou taraudé 12 débouchant perpendiculairement aux axes XX et YY dans l'alésage 11 à partir de celle des surfaces 7 qui est la plus éloignée de l'alésage 11. Cet agencement permet le vissage d'une vis 13 sur une longueur convenable dans le trou 12 pour bloquer la tige 2 dans le corps 6 en rotation et en translation.

Bien entendu, la géométrie du corps 6 décrite et représentée aux figures 1 à 7 n'est fournie qu'à titre d'exemple et peut être sensiblement différente.

La lame 9 est monopièce avec le corps 6 et prolonge latéralement celui-ci, un trou 14 de passage d'une vis d'ancrage osseux telle que 3 étant réalisé dans cette lame 9. Ce trou 14 présente avantageusement, à partir de son bord d'entrée, une surface conique 15 qui s'étend sur la plus grande partie du trou 14, et est prolongé par une surface cylindrique 16. Ainsi, une vis 3 pourvue d'une tête conique 17 adaptée à la surface conique 15 peut venir prendre appui sur cette dernière en fin de vissage dans l'os 4 (figure 4), tandis que la partie lisse 3a de la tête 3 s'appuie sur la surface cylindrique 16.

La lame 9 présente deux surfaces latérales 18 planes sensiblement parallèles qui s'étendent de part et d'autre d'un plan P contenant l'axe XX de l'alésage 11 et parallèle à ces deux surfaces 18. Ces dernières sont raccordées par des congés 19, aux surfaces contiguës 7 du corps 6.

La lame 9 s'étend de manière asymétrique par rapport au plan P, la surface latérale 18a opposée au bord d'entrée du trou 14 étant plus proche du plan P que l'autre surface latérale 18b.

Par ailleurs, un chanfrein 21 est ménagé sur la partie terminale de la lame 9 reliant les deux surfaces latérales 18a, 18b. Le chanfrein 21 est incliné d'un angle A sur la surface latérale 18a la plus proche du plan P et raccordé à celle-ci, cet angle A pouvant être avantageusement, mais non limitativement, de 30° environ.

Des arrondis 22 sont formés sur les côtés opposés du corps 6 situés de part et d'autre de l'alésage 11 ainsi que de part et d'autre du trou fileté 12.

Pour mettre en place le connecteur 1, on l'enfile sur une extrémité de la tige à aspérités 2 (figure 6), laquelle est à cet effet au préalable légèrement écartée de l'os 4. Les arrondis 22 permettent d'enfiler la tige 2 dans l'alésage 11 en évitant d'endommager la surface osseuse.

Lorsque le connecteur 6 est positionné à l'endroit voulu sur la tige 2, le chirurgien le fait pivoter autour de celle-ci jusqu'à ce que son chanfrein terminal 21 soit placé en appui sur la surface osseuse (figure 4). La vis 3 est alors introduite dans le trou 14 et progressivement vissée dans l'os, avec une inclinaison liée à la position angulaire de la lame 9 par rapport à la surface de l'os 4. En fin de vissage, la totalité de la partie filetée 3a et la plus grande partie de la partie lisse 3b sont ancrées dans l'os, tandis que la tête conique 17 en appui sur le siège conique 15 est noyée à l'intérieur de la lame 9.

La réalisation du chanfrein 21 avec une inclinaison convenable sur la surface latérale 18a présente donc l'avantage de fournir à la lame 9 une surface d'appui sur l'os 4, qui permet de positionner angulairement le connecteur de manière plus satisfaisante que les connecteurs antérieurs par rapport à l'os 4, et ce avec une meilleure stabilité.

Il convient de préciser que lorsque la vis 3 subit, en service, des efforts tendant à l'extraire de l'os 4 et donc à faire basculer le connecteur 1 autour de la tige 2. Ces efforts présentent une composante radiale qui tend à appliquer plus fermement le connecteur 1 sur la tige 2, renforçant ainsi la stabilité de l'ensemble et donc la solidité de l'ancrage osseux de la vis 3.

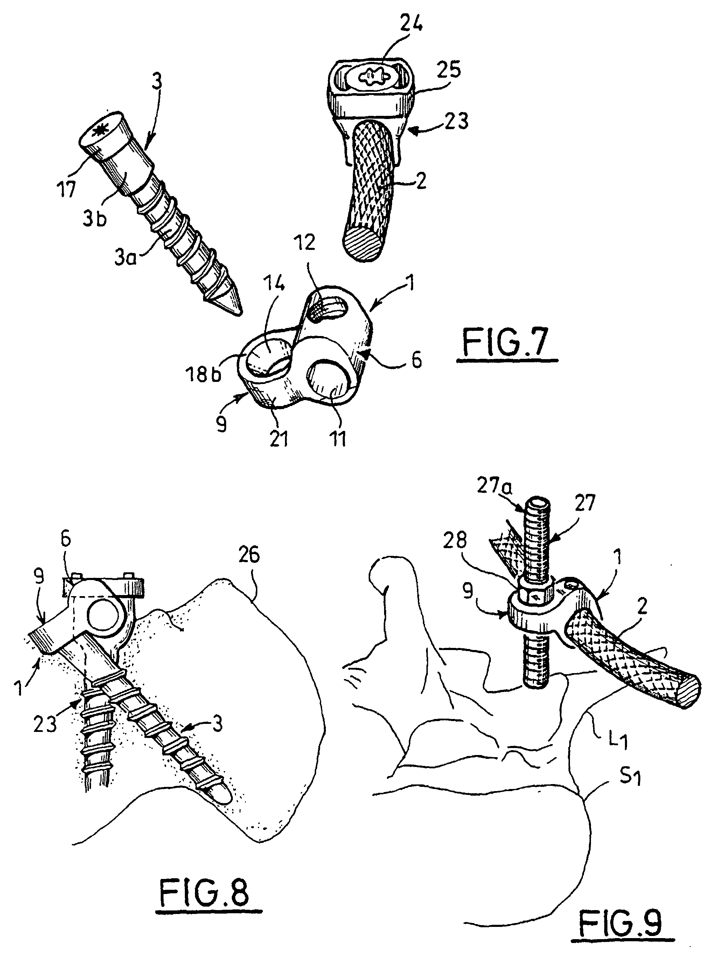

Les figures 7 et 8 représentent une fixation sacrée avec le connecteur 1 selon l'invention, la tige à aspérités 2 étant équipée, au voisinage du connecteur 1, d'une vis d'ancrage 23 pourvue d'un bouchon fileté 24 de fixation et d'une bague 25 (dispositif connu sous la marque "CCD").

Les deux vis 23 et 3 sont enfoncées dans le sacrum 26 avec des orientations angulaires différentes. La méthode de pose mise en oeuvre est généralement la suivante :
- on introduit une vis 23 dans la vertèbre S1 et on place la tige 2 dans cette vis ;
- on enfile le connecteur 1 sur la tige 2 ;
- on introduit une vis 3 d'ancrage osseux dans le trou 14 du connecteur 1 avec une angulation d'environ 30°, correspondant à l'angle A du chanfrein 21 sur la surface latérale 18a ;
- on fixe la tige 2 dans la vis 23 ;
- on applique une compression en rapprochant le connecteur 1 de la vis 23 ;
- on serre le bouchon ou vis 13 du connecteur 1.

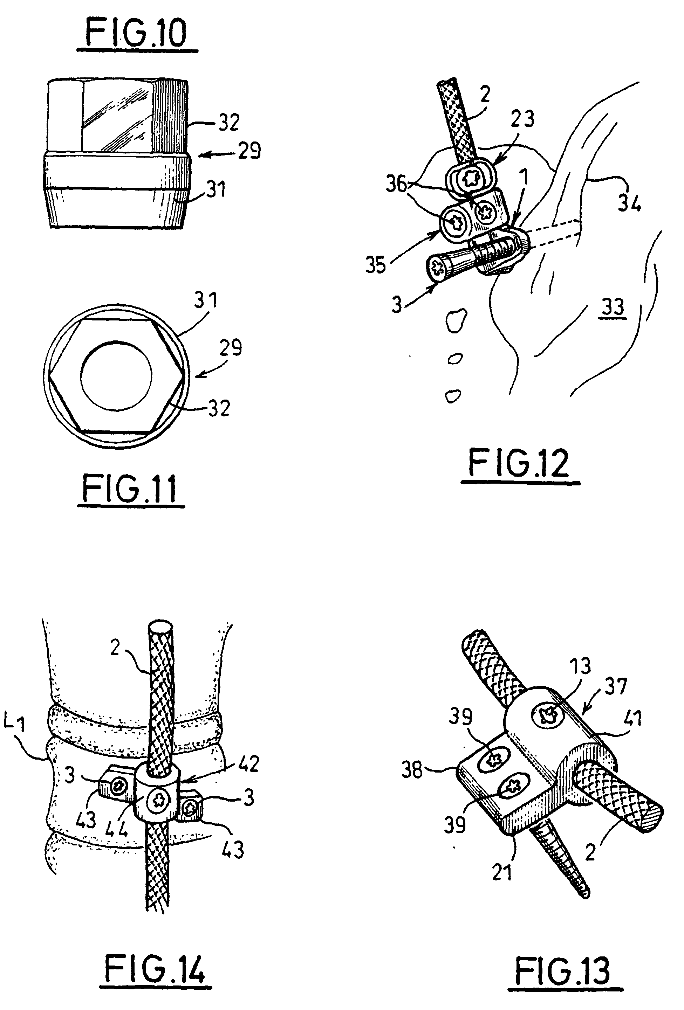

La figure 9 illustre le montage du connecteur 1 dans une fixation lombaire, dans le cas de la réduction d'un spondylolisthèsis. Dans ce cas, le trou 14 de la lame 9 est traversé par une vis 27 à double filetage, d'un type connu en soi, qui vient s'ancrer dans la vertèbre lombaire L1, un écrou 28 serré sur la partie filetée extérieure 27a de la vis 27 venant se bloquer sur la lame 9. Comme dans les montages précédents, le corps 6 est traversé par la tige à aspérités 2 et bloqué sur celle-ci par un bouchon fileté 13.

L'écrou 28 peut être remplacé par un écrou conique 29 (figures 10 et 11), comportant une extrémité conique 31 adaptée pour venir prendre appui sur le siège conique 15, et une partie cylindrique 32 qui fait saillie à l'extérieur du trou 14.

La figure 12 illustre la mise en oeuvre du connecteur 1 selon l'invention pour une fixation ilio-sacrée, la vis 3 d'ancrage du connecteur étant vissée dans l'os iliaque 33, à la base de la crête iliaque 34. La tige d'ostéosynthèse 2 est ici fixée au sacrum par une vis 23 du type "CCD" ainsi que par un sabot 35 pourvu de vis 36.

Dans la forme de réalisation illustrée à la figure 13, le connecteur 37 comporte une lame 38 dont la largeur est augmentée par rapport à celle de la lame 9 de la réalisation précédente, pour pouvoir y ménager deux trous de réception de vis correspondantes 39 d'ancrage osseux. Le corps 41 du connecteur 37 a alors de préférence la même largeur que la lame 38.

Enfin, la forme de réalisation illustrée à la figure 14 diffère des précédentes par le fait que le connecteur 42 comprend deux lames 43 s'étendant de chaque côté du corps 44. Les deux lames 43 sont décalées l'une par rapport à l'autre dans la direction de l'axe de l'alésage du corps 44. Les lames 43 ne sont donc pas en vis-à-vis, ce qui évite un conflit des deux vis d'ancrage 3 dans le corps vertébral tel que L1 lorsque les vis sont convergentes.

Le connecteur selon l'invention présente des avantages importants :
1° Il est universel car il peut remplacer, dans la gamme actuelle de l'instrumentation connue sous la marque commerciale "CCD", le sabot dit "de Chopin", le connecteur d'extension "de Chopin", et le connecteur pour fixation lombaire à profil en L, rappelé dans l'introduction de la présente description. Ceci constitue un avantage très important, tant au niveau de la fabrication en raison de la réduction du prix de revient de l'instrumentation ainsi obtenue, que pour les utilisateurs, en raison de la simplification ainsi réalisée.
   La réduction du coût du montage est particulièrement importante dans le cas d'une fixation sacrée, en raison du coût de fabrication du sabot antérieur "de Chopin".
2° L'encombrement du connecteur selon l'invention est réduit par rapport au sabot "de Chopin" (FR-A-2657774), ce qui facilite l'installation du montage et offre la possibilité de mettre en place un dispositif de liaison transversale (DLT) au niveau lombo-sacré, ce qui n'était pas possible précédemment.
3° Dans le cas d'une fixation ilio-sacrée, la vis d'ancrage 3 dans l'os iliaque 33 (figure 12) peut être positionnée plus bas par rapport à la crête iliaque 34, que dans le cas d'utilisation d'un sabot "de Chopin", ce qui évite d'être gêné par la crête iliaque 34.
4° La qualité de fixation est améliorée du fait que :
   a) le connecteur est toujours placé en appui par le chanfrein 21 de sa lame contre l'os, sans basculement ni suspension comme dans certains cas avec le sabot "de Chopin",
   b) dans une fixation sacrée, le rapprochement du connecteur vers la vis sacrée 3 crée un effet de blocage de ces deux éléments entre eux, comme déjà exposé ci-dessus, ce qui peut éviter le recul spontané de la vis 3 de fixation,
   c) la distance entre une vis sacrée 3 et la vis 13 de fixation peut être augmentée dans les cas où un bras de levier plus important est nécessaire,
   d) la fixation peut être doublée, au moyen de deux connecteurs équipés chacun de deux vis, tels que des connecteurs 37, 42,
   e) le connecteur permet au chirurgien de choisir l'orientation optimale de la vis 3 d'ancrage en fonction de la largeur du sacrum et de la qualité de l'os.
5° Possibilité de bloquer le connecteur à tout moment de la mise en place du montage, ce qui rend son utilisation plus souple que celle du sabot "de Chopin", et offre la possibilité de tourner la tige 2 pour accentuer une lordose ou pour réduire une scoliose avec fixation au sacrum.
6° Possibilité de poser dans le sacrum une vis à double filetage 27 pour la fixation du connecteur (ce type de vis étant jusqu'à présent utilisée pour la réduction-fixation du spondylolisthèsis).

La méthode chirurgicale de pose du connecteur selon l'invention est la suivante.

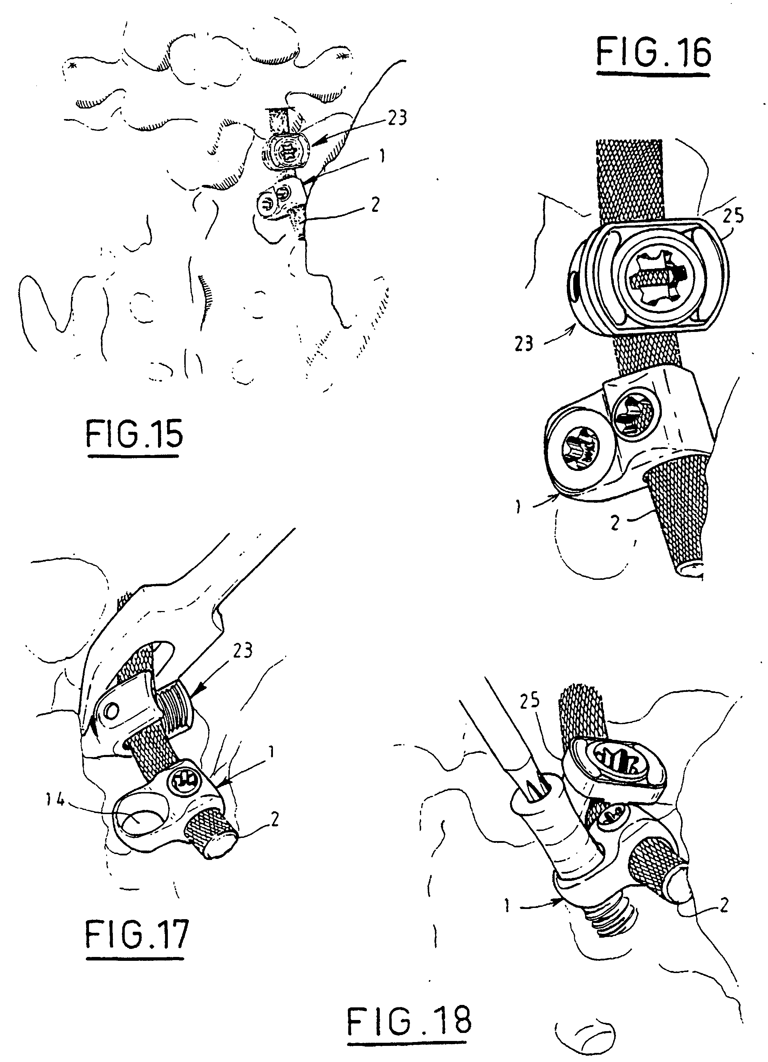

### 1°) Fixation sacrée (Figures 15 à 18)

Le connecteur a été conçu pour simplifier et améliorer la fixation sacrée lors d'une instrumentation rachidienne à tige descendant au sacrum. Il peut être implanté dès le début de l'ostéosynthèse si le sacrum est le point fixe d'une réduction importante, ou en fin d'intervention s'il est considéré comme un point de stabilisation complémentaire. Il est utilisé indifféremment à droite ou à gauche.

Le connecteur intervient en complément d'une vis pédiculaire de la première pièce sacrée. Si son usage isolé peut se concevoir, il n'est pas conseillé. La distance le séparant de la vis pédiculaire peut être augmentée sans compliquer sa pose. Plusieurs connecteurs peuvent se suivre sur la tige.

La pose du connecteur sous-entend deux étapes :
- le choix et le travail de la tige,
- la fixation de l'implant à l'os et à la tige.

### a) La tige :

Le choix de longueur de la tige devra tenir compte des 1,5 à 2 cm nécessaires à la pose d'un connecteur, ou plus si on désire augmenter le bras de levier ou poser deux connecteurs.

La tige devra être cintrée pour épouser la face postérieure du sacrum qui est en général plate. Ce cintrage doit prévoir le passage de la tige dans les différents implants lombaires, la vis sacrée avec une remontée sur l'arrière du sacrum.

La tige choisie sera équipée du connecteur à sa partie distale, fixé provisoirement dans le plan frontal d'implantation, sa lame dirigée vers le dedans, et l'écrou de serrage sur la tige en arrière. La vis S1 et/ou les autres sont alors fermées par leur bouchon-pastille. L'utilisation des instruments d'introduction permet de mettre au fond du corps de la vis la tige, et d'appliquer la tige sur l'os du sacrum (Figure 17).

### b) Fixation du connecteur :

Quand la tige est dans sa position définitive, le connecteur peut être fixé au sacrum. Le connecteur est libéré de la tige en dévissant la vis de serrage. Il faut d'abord choisir son site d'implantation sur l'axe de la tige, plus ou moins près de la vis S1. Puis il faut choisir l'orientation de la vis latérale de fixation. En général, cette vis fera 30 à 40° avec la verticale antéro-postérieure, mais cet angle peut varier en fonction de l'anatomie, de la latéralité de la tige et des données du scanner par exemple. Le chirurgien pourra adapter sa technique de fixation aux constatations per opératoires, en diminuant ou en augmentant cet angle afin d'avoir une vis dont la prise osseuse est assurée (Figure 18).

Le choix du site d'implantation du connecteur et de l'angle de visée impose le point de pénétration osseuse. Il se situe le plus souvent au bord externe du 1er trou sacré. S'il était trop médian il suffirait de cintrer la tige in situ pour retrouver l'endroit adéquat. Si la bonne orientation n'est pas possible parce que la rotation du connecteur est empêchée par la corticale postérieure du sacrum, il suffit d'enlever un peu d'os au ciseau frappé pour obtenir la rotation voulue et un bon contact osseux.

A travers l'orifice de la lame du connecteur, l'os est attaqué à la pointe carrée. On peut s'aider du cylindre à extrémité dentée du viseur sacré de l'instrumentation CD. Celui-ci est impacté au marteau. On traverse alors l'os comme habituellement à la mèche ou à la curette, de préférence jusqu'à la corticale antérieure. Il est dangereux de forcer l'orientation de la visée avec une mèche car celle-ci peut casser et fuir en avant, imposant une extraction périlleuse et difficile. La longueur de l'orifice déterminée (en général 40 à 50 mm), celui-ci est taraudé. Une vis de 7 mm est vissée à fond, appliquant le connecteur sur le sacrum. La tenue est en général excellente.

La pose du connecteur se termine par la mise en compression du connecteur et de sa vis sur la vis pédiculaire en S1. Pour ce faire, on utilise la pince à compression, placée de part et d'autre de la vis S1 et du connecteur. L'écrou de serrage orienté en haut et en arrière est alors facilement serré. La fixation sacrée est alors terminée. Elle s'entend par définition bilatérale.

### 2°) Fixation iliaque :

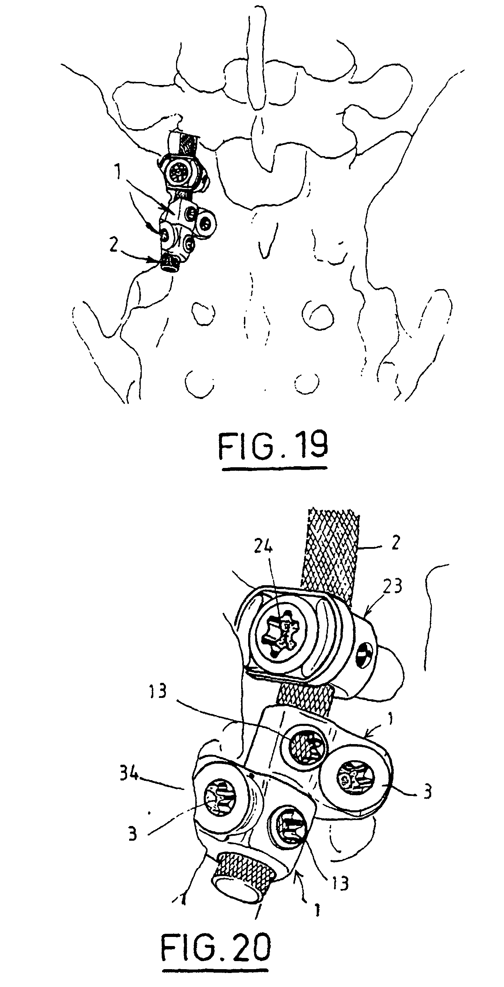

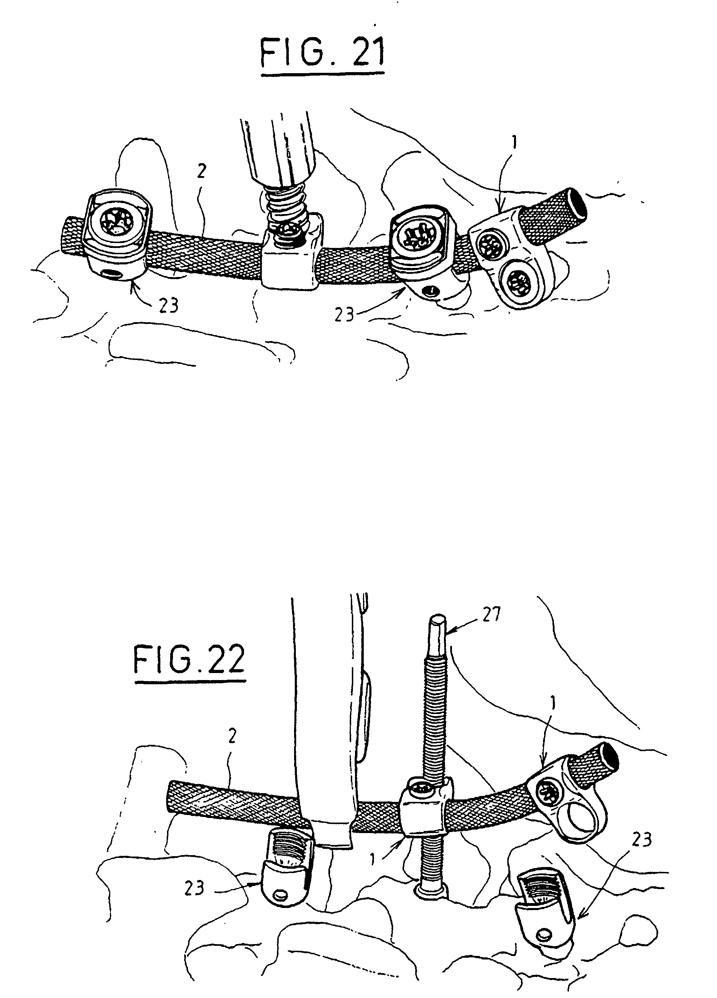

Quand une fixation supplémentaire est nécessaire, le connecteur peut être utilisé pour une prise iliaque. Il est en fin de montage, enfilé sur une tige plus longue qu'à l'ordinaire, oeillet vers le haut et l'écrou de serrage vers le dedans. Il est donc dans une position inverse à celle de la prise sacrée. La lame du connecteur est appuyée sur la crête iliaque. A travers son orifice l'os est attaqué à la pointe carrée puis à la curette. La pénétration est intercorticale. Une longue vis (50 mm ou plus) assurera la fixation iliaque. Le connecteur est verrouillé sur la tige par serrage de l'écrou (Figures 19 et 20). Cette fixation pourra facilement être démontée après la prise de l'arthrodèse pour libérer l'articulation sacro-iliaque.

### 3°) Fixation lombaire décalée :

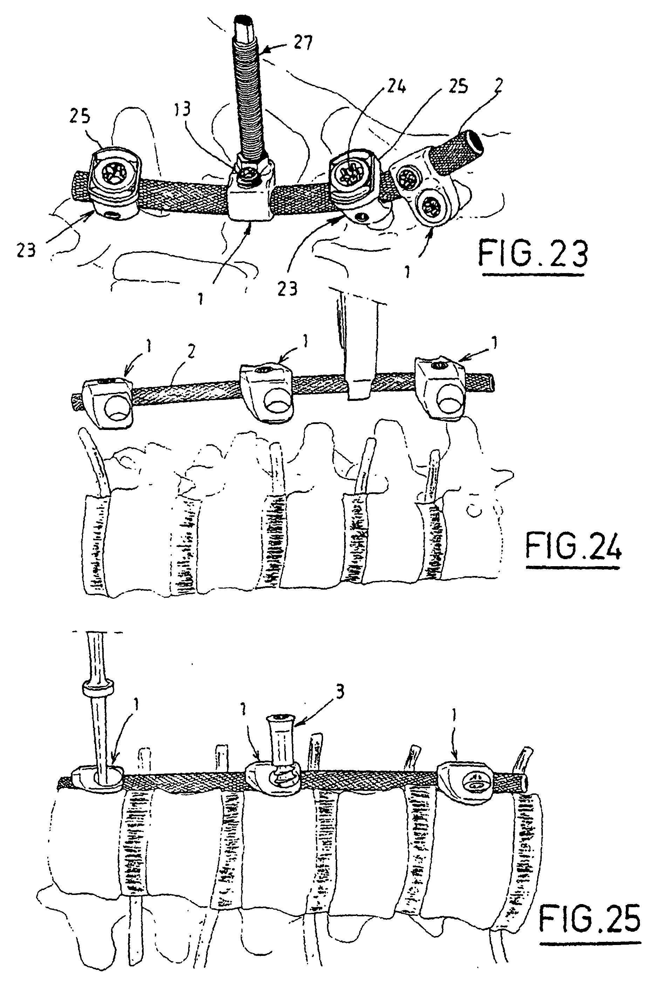

Il est parfois nécessaire d'obtenir une fixation pédiculaire décalée par rapport à l'axe de la tige (Figure 23). Cette fixation est soit voulue et programmée comme c'est le cas dans la chirurgie du spondylolisthésis, soit elle est parfois imposée par le non alignement des points d'entrée des vis. Dans cette dernière situation, le connecteur intervient comme un élément de sauvetage.

Le connecteur s'utilise avec des vis 27 à double filetage. La partie arrière de la vis pénètre dans le trou 14 de la lame du connecteur 1 et un écrou permet la solidarisation de l'ensemble avec une liaison à 90° double filetage connecteur et vis. L'important est que le serrage du connecteur à la vis laisse libre le connecteur sur la tige, ce qui permet un ajustement de correction.

Le connecteur doit être enfilé sur la tige avant la pose de celle-ci. Deux cas de figure sont possibles.

### a) La vis à double filetage est déjà en place :

La tige habillée du connecteur est descendue dans les différents implants, l'oeillet 14 du connecteur recevant le double filetage de la vis 27 (Figure 22). L'écrou aide à la descente de la vis et les implants adjacents sont fermés. Différents efforts en compression ou en distraction peuvent être faits avant de verrouiller le connecteur sur la tige.

### b) La vis à double filetage n'est pas encore en place :

La tige habillée du connecteur est descendue dans les implants. Son glissement sur la tige va le positionner en face du pédicule (Figure 21).

Soit la visée pédiculaire a déjà été faite et il suffit de mettre la vis à double filetage à travers l'oeillet du connecteur. Secondairement l'écrou sera descendu et les efforts de correction effectués. Soit la visée pédiculaire n'a pas été faite et le pédicule sera alors "cathétérisé" à travers l'oeillet, c'est-à-dire qu'on ajuste la position de l'oeillet du connecteur par rapport à l'axe du pédicule et la vis sera placée comme précédemment.

### 4°) Fixation antérieure :

Le connecteur à un ou deux oeillets dans sa lame peut être utilisé en chirurgie par voie antérieure. Il est alors fixé sur la face latérale des corps vertébraux.

L'abord nécessite une ligature des vaisseaux lombaires et/ou intercostaux. La partie médiane de la face latérale des corps vertébraux est dégagée. Il faut alors choisir une tige de longueur adéquate et la cintrer pour que, appliquée sur le rachis, elle relie les parties médianes des corps. On habille la tige du nombre de connecteurs voulus, c'est-à-dire un par corps vertébral et on la pose sur la face latérale du rachis (Figure 24), convexité en avant au niveau lombaire, concavité en avant au niveau dorsal.

Si on pense que les connecteurs sont trop encombrants, il faut soit placer l'instrumentation plus en arrière, soit creuser le corps vertébral pour enfouir le connecteur et la tige. A travers les oeillets des connecteurs tous vers l'avant ou vers l'arrière, il est facile de forer les corps vertébraux et de mettre en place des vis "sacrées" 3 qui ont une excellente tenue dans le corps vertébral (Figure 25).

Tous les connecteurs étant fixés à l'os, toutes les manoeuvres de correction sont possibles : distraction, compression, rotation ou cintrage in situ de la tige. La réduction une fois obtenue, les connecteurs sont verrouillés sur la tige.

## Revendications

1. Connecteur (1; 37; 42) pour instrumentation d'ostéosynthèse rachidienne, destiné à une fixation lombaire, ou sacrée ou ilio-sacrée, adapté pour solidariser une tige d'ostéosynthèse (2) avec une vis (3) d'ancrage osseux, comportant un corps (6; 41, 44) percé d'un alésage cylindrique (11) de passage de ladite tige et d'un trou taraudé (12) débouchant perpendiculairement dans cet alésage, ce trou étant adapté pour recevoir un élément (13) de fixation de la tige au connecteur, lequel comprend en outre au moins une lame (9; 38; 43) monopièce avec le corps et prolongeant latéralement celui-ci, au moins un trou (14) de passage d'une vis d'ancrage osseux étant réalisé dans la lame, **caractérisé en ce que** la lame (9) présente deux surfaces latérales planes (18a, 18b) sensiblement parallèles qui s'étendent de part et d'autre d'un plan (P) contenant l'axe (XX) de l'alésage (11) et parallèle à ces deux surfaces latérales, lesquelles sont raccordées par des décrochements (19) aux surfaces contiguës (7) du corps.

2. Connecteur selon la revendication 1, **caractérisé en ce que** la lame (9) s'étend de manière asymétrique par rapport au plan (P) contenant l'axe (XX) de l'alésage (11), la surface latérale (18a) opposée au bord d'entrée du trou (14) d'introduction de la vis (3) étant plus proche dudit plan que l'autre surface latérale (18b).

3. Connecteur selon la revendication 2, **caractérisé en ce qu'**un chanfrein (21) est ménagé sur la partie terminale de la lame (9) reliant ses deux surfaces latérales (18a, 18b) et est incliné d'un angle déterminé (A) sur la surface latérale (18a) la plus proche dudit plan (P) contenant l'axe (XX) de l'alésage (11), ce chanfrein étant raccordé à cette surface latérale (18a).

4. Connecteur selon la revendication 3, **caractérisé en ce que** l'angle (A) du chanfrein (21) avec ladite surface latérale (18a) est de l'ordre de 30 degrés.

5. Connecteur selon la revendication 1, **caractérisé en ce que** des arrondis (22) sont formés sur les côtés opposés du corps (6) situés de part et d'autre de l'alésage (11) et du trou fileté (12) de passage dudit élément (13) de fixation.

6. Connecteur (37) selon la revendication 1, **caractérisé en ce que** la lame (38) a une largeur suffisante pour pouvoir y ménager deux trous de réception de deux vis (39) d'ancrage osseux (figure 13).

7. Connecteur (42) selon la revendication 1, **caractérisé en ce qu'**il comprend deux lames (43) s'étendant de chaque côté du corps (44) (figure 14).

8. Connecteur (42) selon la revendication 7, **caractérisé en ce que** les deux lames (43) sont décalées l'une par rapport à l'autre dans la direction de l'axe (XX) de l'alésage (11) du corps (44).

9. Connecteur (42) selon la revendication 1, **caractérisé en ce que** le corps (6) est de forme oblongue en section transversale et son alésage (11) est décalé par rapport à l'axe (XY) du corps (6), parallèlement à cet axe.

## Patentansprüche

1. Verbinder (1; 37; 42) zum Gebrauch für Wirbelsäulenosteosynthese-Instrumente zur Befestigung der Lendenwirbel, des Kreuzbeins oder des Kreuzbeins mit dem Darmbein, welcher angepasst ist, um eine Osteosynthese-Stange (2) mit einer Knochenankerschraube (3) zu verbinden, welcher einen Körper (6; 41, 44) mit einer zylindrischen Durchgangsbohrung (11) für die Stange und eine Gewindebohrung (12) aufweist, die rechtwinklig in die zylindrische Bohrung mündet, wobei diese Bohrung so beschaffen ist, dass sie ein Element (13) für die Befestigung der Stange des Verbinders aufnehmen kann, und die außerdem mindestens eine Öse (9; 38; 43) aufweist, die einstückig mit dem Körper ausgebildet ist und ihn in seitlicher Richtung verlängert, sowie mindestens eine Durchgangsbohrung (14) für eine Knochenankerschraube aufweist, die in der Öse vorgesehen ist,
**dadurch gekennzeichnet, dass**
die Öse (9) zwei im Wesentlichen parallele ebene Seitenflächen (18a, 18b) aufweist, die sich auf beiden Seiten einer Ebene (P) erstrecken, welche die Achse (XX) der zylindrischen Bohrung (11) aufweist und parallel zu den beiden Seitenflächen liegt, welche mittels Absätzen (19) mit den angrenzenden Flächen (7) des Körpers verbunden sind.

2. Verbinder nach Anspruch 1,
**dadurch gekennzeichnet, dass**
die Öse (9) asymmetrisch zu der Ebene (P) verläuft, welche die Achse (XX) der zylindrischen Bohrung (11) einschließt, wobei die gegenüber der Eintrittskante der Bohrung (14) für das Einfügen der Knochenankerschraube (3) liegende Seitenfläche (18a) näher an der Ebene liegt als die andere Seitenfläche (18b).

3. Verbinder nach Anspruch 2,
**dadurch gekennzeichnet, dass**
am Endabschnitt der Öse (9) eine Seitenfase (21) vorgesehen ist, welche die beiden Seitenflächen (18a, 18b) miteinander verbindet und in einem bestimmten Winkel (A) zur Seitenfläche (18a) geneigt ist, welche am nächsten an der Ebene (P) liegt, welche die Achse (XX) der Zylinderbohrung (11) einschließt, und die Seitenfase an die Seitenfläche (18a) angeschlossen ist.

4. Verbinder nach Anspruch 3,
**dadurch gekennzeichnet, dass**
der Winkel (A) zwischen der Seitenfase (21) und der Seitenfläche (18a) etwa 30° beträgt.

5. Verbinder nach Anspruch 1,
**dadurch gekennzeichnet, dass**
an den gegenüberliegenden Seiten des Körpers (6) Rundungen (22) vorgesehen sind, welche auf beiden Seiten der zylindrischen Bohrung (11) und der Gewindebohrung (12) für den Durchgang des Befestigungselementes (13) angeordnet sind.

6. Verbinder (37) nach Anspruch 1,
**dadurch gekennzeichnet, dass**
die Öse (38) eine ausreichende Weite hat, die es erlaubt, zwei Bohrungen für die Aufnahme von zwei Knochenankerschrauben (39) einzubringen (Figur 13).

7. Verbinder (42) nach Anspruch 1,
**dadurch gekennzeichnet, dass**
er zwei Ösen (43) aufweist, die sich auf beide Seiten des Körpers (44) erstrecken (Figur 14).

8. Verbinder (42) nach Anspruch 7,
**dadurch gekennzeichnet, dass**
die beiden Ösen (43) zueinander versetzt in der Richtung der Achse (XX) der zylindrischen Bohrung (11) des Körpers (44) angeordnet sind.

9. Verbinder (42) nach Anspruch 1,
**dadurch gekennzeichnet, dass**
der Körper (6) im Querschnitt eine längliche Form hat, und dass seine zylindrische Bohrung (11) bezüglich der Achse (XY) des Körpers (6) parallel zu dieser Achse versetzt angeordnet ist.

## Claims

1. Connector (1; 37; 42) for spinal osteosynthesis equipment intended for lumbar, sacral or iliosacral fixing, suitable for making an osteosynthesis rod (2) integral with a bone anchoring screw (3), consisting of a body (6; 41; 44) with a cylindrical drilling (11) for the said rod to pass through and a tapped hole (12) emerging perpendicularly in this bore, this hole being suitable to take a fastener (13) for fixing the rod to the connector, which comprises in addition at least one plate (9; 38; 43), one-piece with the body and extending the latter laterally, at least one hole (14) being made in the plate for a bone anchoring screw to pass through, **characterised in that** the plate (9) has two more or less parallel flat lateral surfaces (18a, 18b) which extend on either side of a plane (P) containing the centreline (XX) of the bore (11) and parallel to these two lateral surfaces which are connected by disconnectors (19) to the adjacent surfaces (7) of the body.

2. Connector according to Claim 1, **characterised in that** the plate (9) extends asymmetrically compared with the plane (P) containing the centreline (XX) of the bore (11), the lateral surface (18a) opposite to the inlet edge of the hole (14) for inserting the screw (3) being closer to the said plane than the other lateral surface (18b).

3. Connector according to Claim 2, **characterised in that** a chamfer (21) is made on the end of the plate (9) connecting its two lateral surfaces (18a, 18b) and is inclined at a particular angle (A) to the nearest lateral surface (18a) of the said plane (P) containing the centreline (XX) of the bore (11), this chamfer being connected to this lateral surface(18a).

4. Connector according to Claim 3, **characterised in that** the angle (A) of the chamfer (21) with the said lateral surface (18a) is in the region of 30 degrees.

5. Connector according to Claim 1, **characterised in that** rounded parts (22) are formed on the opposite sides of the body (6) located on either side of the bore (11) and of the threaded hole (12) for the said fastener (13) to pass through.

6. Connector (37) according to Claim 1, **characterised in that** the plate (38) is wide enough for two holes to be made in it to take two bone anchoring screws (39) (Figure 13).

7. Connector (42) according to Claim 1, **characterised in that** it comprises two plates (43) extending on each side of the body (44) (Figure 14).

8. Connector (42) according to Claim 7, **characterised in that** the two plates (43) are offset to each other in the direction of the centreline (XX) of the bore (11) of the body (44).

9. Connector (42) according to Claim 1, **characterised in that** the body (6) is oblong in cross section and its bore (11) is offset in relation to the centreline (XY) of the body (6), parallel to this centreline.
